# EUROPÄISCHE PATENTSCHRIFT

(11) **EP 3 430 890 B1**
(45) Veröffentlichungstag und Bekanntmachung des Hinweises auf die Patenterteilung: **25.11.2020**
(21) Anmeldenummer: 18184483.8
(22) Anmeldetag: 19.07.2018
(51) Int. Cl.: A01K 1/01, A01K 67/033, C05F 17/00, A01K 29/00

(54) **VORRICHTUNG, ANLAGE UND VERFAHREN ZUR RESTSTOFF-VERWERTUNG IN DER NUTZTIERHALTUNG**
DEVICE, SYSTEM AND METHOD FOR RECYCLING LEFTOVER MATERIALS IN LIVESTOCK FARMING
DISPOSITIF, INSTALLATION ET PROCÉDÉ DE VALORISATION DES RÉSIDUS DANS L'ÉLEVAGE DE BÉTAIL

(30) Priorität: 21.07.2017 DE 202017104380 U
(43) Veröffentlichungstag der Anmeldung: 23.01.2019
(73) Patentinhaber: Big Dutchman International GmbH, 49377 Vechta (DE)
(72) Erfinder: Heetjans, Kai, 49846 Hoogstede (DE)
(74) Vertreter: Eisenführ Speiser

(56) Entgegenhaltungen:
- WO-A1-01/32586
- WO-A1-2008/134865
- US-A- 4 262 633
- US-A- 6 001 146

## Beschreibung

Die Erfindung betrifft eine Vorrichtung, eine Anlage und ein Verfahren zur Reststoff-Verwertung in der Nutztierhaltung.

Die Reststoff-Verwertung, insbesondere die Verwertung von Tierkot, in der Nutztierhaltung stellt eine Herausforderung dar, insbesondere hinsichtlich der Kosten und der Reduktion von Emissionen und Schadstoffen. Aus der WO 2012/115959 A2, WO 2007/05937 A1, RU 2 285 400 C2 und der EP 2 703 372 A1 ist es bekannt, in der Reststoff-Verwertung in der Nutztierhaltung Larven einzusetzen. Allerdings haben sich existierende Lösungen nicht in der Praxis durchgesetzt. Insbesondere für die Nutztierhaltung in großem Maßstab in Massenställen haben sich existierende Lösungen nicht bewährt, insbesondere aufgrund mangelnder Prozesssicherheit.

Die US 6 001 146 A offenbart eine Vorrichtung und ein Verfahren zur kontinuierlichen Behandlung von Fäulnisabfällen, bei denen der Abfall von Fliegenlarven gefressen wird. Die WO 01/32586 A1 offenbart eine Vorrichtung zur kontinuierlichen Bioumwandlung von Fäulnisabfällen umfassend eine Entsorgungsbahn mit seitlichen Seitenwänden und einem Boden zur Aufnahme des Entsorgungsvolumens. Die US 4 262 633 A offenbart ein integriertes Abfallverwertungs- und -verarbeitungssystem, das biologisch abbaubare flüssige und feste Abfälle in Geflügelprodukte (oder dergleichen) und humusähnliche Substanzen umwandelt.

Es ist daher eine Aufgabe der vorliegenden Erfindung, eine Vorrichtung, eine Anlage und ein Verfahren zur Reststoff-Verwertung in der Nutztierhaltung bereitzustellen, welche einen oder mehrere der genannten Nachteile verbessern und/oder eine verbesserte Vorrichtung, eine verbesserte Anlage und ein verbessertes Verfahren zur Reststoff-Verwertung in der Nutztierhaltung bereitzustellen.

Diese Aufgabe wird gelöst durch eine Vorrichtung zur Reststoff-Verwertung in der Nutztierhaltung, umfassend eine Fördervorrichtung zum Fördern von Tierkot entlang einer Förderstrecke, wobei die Förderstrecke einen Larveneinbringabschnitt zum Zudosieren von Larven und/oder Larveneiern, einen Koteinbringabschnitt zum

Zudosieren von Tierkot in Abhängigkeit von einem oder mehreren Prozessparametern und einen Larvenseparierabschnitt zum Separieren der Larven aufweist.

Die Erfindung beruht unter anderem auf der Erkenntnis, dass eine besonders vorteilhafte Lösung erzielt werden kann, wenn der Tierkot von einer Fördervorrichtung entlang einer Förderstrecke gefördert wird. Bevorzugt ist es, dass diese Förderung des Tierkots erfolgt, während der Tierkot mit Larven und/oder Larveneiern versetzt wird und sich diese vom Tierkot ernähren, darin wachsen und dadurch die Menge an Tierkot reduzieren. Es ist daher ebenfalls besonders vorteilhaft, dass die Vorrichtung zur Reststoffverwertung in der Nutztierhaltung einen Koteinbringabschnitt aufweist, über den zusätzlicher Tierkot auf die Fördervorrichtung zudosiert werden kann. Das Larvenwachstum ist unter anderem abhängig von der Verfügbarkeit von ausreichend Nahrung für die Larven und/oder Larveneier, insbesondere in Form von Tierkot, insbesondere in einer ausreichenden Menge und Beschaffenheit. Beispielsweise in Abhängigkeit von der Länge der Förderstrecke und/oder der Menge und/oder Beschaffenheit des anfangs eingebrachten Kots und/oder der Menge und/oder Art der eingebrachten Larven und/oder Larveneier muss unterschiedlich viel Tierkot im Koteinbringabschnitt zudosiert werden, um ein möglichst gutes Larvenwachstum zu ermöglichen.

Tierkot kann vorzugsweise auch als Substrat bezeichnet werden und insbesondere mit zusätzlichen Stoffen, beispielsweise Nährstoffen, versetzt sein oder werden.

Vorzugsweise erfolgt auch die Zudosieren von Larven und/oder Larveneiern in Abhängigkeit von einem oder mehreren Prozessparametern, wie Länge der Förderstrecke und/oder der Menge und/oder Beschaffenheit des anfangs eingebrachten Kots und/oder der Menge und/oder Art des zuzudosierenden Tierkots.

Besonders bevorzugt ist das Zudosieren von Junglarven, insbesondere im Alter von etwa vier Tagen. Vorzugsweise werden die Junglarven aus Larveneiern vorgezogen, insbesondere etwa 4 bis 7 Tage. Während dieser Phase erfolgt der Larvenschlupf und vorzugsweise eine Fütterung, beispielsweise mit "Gainesville diet" oder äquivalentem 'leicht verdaulichem' Substrat. Das Zudosieren von Junglarven hat gegenüber dem Zudosieren von Larveneiern Vorteile, da das direkte Zudosieren von Eiern zu schlechtem Schlupf und schlechtem Junglarvenüberleben führen kann. Vorzugsweise umfasst die Vorrichtung zur Reststoff-Verwertung in der Nutztierhaltung eine Larvenaufzuchteinrichtung, in der die Junglarven aus Larveneiern vorgezogen werden können, wie hier beschrieben.

Im Larvenseparierabschnitt werden die Larven separiert, insbesondere erfolgt dort eine Trennung der Larven vom Restkot, also insbesondere dem Anteil des Tierkots, der von den Larven nicht aufgenommen wurde. Wenn die Larven weiter verwendet werden, wird in diesem Zusammenhang auch oft von Larvenernte gesprochen. Der Larvenseparierabschnitt wird daher auch als Ernteabschnitt bezeichnet.

Die Fördervorrichtung ist vorzugsweise als Bandförderer ausgebildet und/oder umfasst vorzugsweise einen oder mehrere Bandförderer. Ein solcher Bandförderer weist vorzugsweise ein Förderband, das auch als Aufzuchtband bezeichnet werden kann, auf und ist vorzugsweise mit Endlosband mit Ober- und Untertrum ausgebildet, wobei der Tierkot vorzugsweise auf dem Obertrum gefördert wird. Die Fördervorrichtung ist vorzugsweise angetrieben, insbesondere elektrisch. Die Fördervorrichtung kann ganz oder teilweise in einer Kottrocknungsvorrichtung angeordnet sein oder ganz oder teilweise eine solche Kottrocknungsvorrichtung bilden. Eine Kottrocknungsvorrichtung ist vorzugsweise mehretagig ausgebildet und umfasst meist zwei oder mehrere Teilförderstrecken übereinander, die in der Regel gegenläufige Förderrichtungen aufweisen und vorzugsweise durch mehrere übereinander angeordnete Bandförderer gebildet sind. Kottrocknungsvorrichtungen sind in der Regel belüftet, um eine gute und schnelle Trocknung des Kots zu ermöglichen.

Eine Weiterbildung der Fördervorrichtung kann auch als Plattenförderer ausgebildet sein und eine Mehrzahl von Platten, die vorzugsweise siebartig perforiert sind, umfassen. Ein solcher Plattenförderer ist vorzugsweise als Endlosförderer ausgelegt, wobei die einzelnen Platten ferner vorzugsweise durch einen Trieb, insbesondere einen Kettentrieb, angetrieben werden. Die Platten sind vorzugsweise an dem Kettentrieb an mindestens einer Längsseite befestigt. Die Befestigung kann drehbar gestaltet sein, so dass die Platten gelenkig mit dem Kettentrieb verbunden sind. Der Kettentrieb ist vorzugsweise angetrieben, insbesondere elektrisch.

Es ist bevorzugt, dass die Fördervorrichtung belüftet ist und/oder belüftbar ausgestaltet ist und/oder eine Belüftungsvorrichtung aufweist und/oder mit einer Belüftungsvorrichtung verbindbar ist.

Die Fördervorrichtung, insbesondere ein Förderband der Fördervorrichtung, weist vorzugsweise eine Perforation auf. Die Perforation ist vorzugsweise kleiner als 2 mm im Durchmesser, so dass die Larvenköpfe oder -körper während des Transports und Wachstums nicht darin stecken bleiben können. Weiter bevorzugt ist die Fördervorrichtung, insbesondere ein Förderband der Fördervorrichtung, geschlossen ausgeführt.

Die Vorrichtung zur Reststoff-Verwertung in der Nutztierhaltung ist vorzugsweise ausgebildet, Junglarven bis zum Stadium der Vorpuppe in dem Tierkot zu belassen, insbesondere etwa bis zu einem Alter von 13 bis 15 Tagen.

In einer bevorzugten Ausführungsform umfasst die Vorrichtung eine Separiervorrichtung mit einem Larvenauffangbehälter und/oder einen Restkotauffangbehälter. Eine bevorzugte Weiterbildung zeichnet sich dadurch aus, dass die Separiervorrichtung eine Lichtquelle und/oder eine Larvenbrücke und/oder eine mechanische Trennvorrichtung aufweist.

Es ist ferner bevorzugt, dass die Lichtquelle angeordnet und/oder ausgebildet ist, den Larvenseparierabschnitt der Förderstrecke zu beleuchten. Vorzugsweise ist die Lichtquelle parallel zur Längserstreckungsrichtung des Larvenseparierabschnitts angeordnet.

Vorzugsweise ist die Larvenbrücke als Vorrichtung ausgebildet, über die Larven von der Förderstrecke in den Larvenauffangbehälter gelangen, vorzugsweise durch Eigenbewegung, können. Die Larvenbrücke kann auch als Ernterampe bezeichnet werden.

Vorzugsweise ist die Larvenbrücke derart ausgebildet, dass sie ein Gefälle in Richtung der Fördervorrichtung, insbesondere in Richtung des Förderbands, aufweist.

Ferner vorzugsweise weist die Larvenbrücke einen Absenkmechanismus auf, mit dem ein der Fördervorrichtung, insbesondere dem Förderband, zugewandtes Ende der Larvenbrücke in Richtung der Fördervorrichtung, insbesondere des Förderbands, abgesenkt werden kann.

Es ist ferner bevorzugt, dass eine oder mehrere Kanten der Larvenbrücke, insbesondere die Kanten, die von Larven bzw. Vorpuppen zu passieren sind, verrundet sind.

Ferner vorzugsweise weist die Vorrichtung zur Reststoff-Verwertung in der Nutztierhaltung ein Vorpuppentransportband, das auch als Larvensammelband bezeichnet werden kann, auf, auf das die Larven bzw. Vorpuppen nach dem Verlassen der Larvenbrücke und/oder des Larvenauffangbehälters gelangen und auf dem sie weitertransportiert werden können.

Das Larvensammelband ist vorzugsweise ein Endlos-Förderband aus einem strukturierten Material, bspw. aus gewebter Herstellung, noch weiter bevorzugt hergestellt aus Polypropylen.

Durch die Strukturierung finden die Vorpuppen einen guten Halt und es kommt nicht zu ungewollten Stauungen oder Ansammlungen. Ein weiterer Vorteil ist die Unpolarität des Materials, wodurch eine einfache Reinigung mittels Bürste möglich wird.

Vorzugsweise ist das Larvensammelband in einem Larvenkanal angeordnet. Der Larvenkanal ist vorzugsweise im Querschnitt als U-förmiges Profil ausgeführt, wobei die Öffnung des U-Profils vorzugsweise nach oben gerichtet ist und weiter bevorzugt mit zusätzlichen Abkantungen an den Schenkelenden, am oberen Ende, welche nach innen gerichtet sind.

Gemäß einer bevorzugten Ausführungsform ist vorgesehen, dass der Larvenauffangbehälter, insbesondere ein Inneres des Larvenauffangbehälters, derart angeordnet ist, dass er vor Licht der Lichtquelle der Separiervorrichtung geschützt sind. Insbesondere ist es bevorzugt, dass der Larvenauffangbehälter, insbesondere Inneres des Larvenauffangbehälters, derart angeordnet ist, dass er nicht von der Lichtquelle der Separiervorrichtung beleuchtet wird und/oder vor Licht, insbesondere ausgehend von der Lichtquelle der Separiervorrichtung, geschützt ist und/oder abgedunkelt und/oder in einem abgedunkelten Bereich angeordnet ist. Bevorzugt ist es, dass der Larvenauffangbehälter, insbesondere Inneres des Larvenauffangbehälters, derart angeordnet ist, dass er weniger Licht empfangen und/oder empfangen kann als die Fördervorrichtung im Bereich des Larvenseparierabschnitts.

Erfindungsgemäß ist vorgesehen, dass die Lichtquelle angeordnet und/oder ausgebildet ist, die Larvenbrücke zu beleuchten. Vorzugsweise ist vorgesehen, dass die Lichtquelle angeordnet und/oder ausgebildet ist, die Fördervorrichtung im Larvenseparierabschnitt von der Beleuchtung weitgehend auszunehmen.

Es wurde erkannt, dass die Larven, sobald sie sich zu Vorpuppen entwickelt haben, einen Sinn für Licht besitzen. Und zwar tritt der Effekt ein, dass die Vorpuppen sich von Licht angezogen fühlen. Vorzugsweise sollte die Lichtquelle ausgebildet sein, eine tagesähnliche Lichtfarbe abzugeben, vorzugsweise weißes Licht und/oder kaltweißes Licht und/oder ggfs. mit blauen und/oder UV-Anteilen und/oder IR-Strahlung. Vorzugsweise ist in dem Ernteabschnitt eine Beleuchtung außerhalb der Fördervorrichtung, insbesondere außerhalb eines Förderbands, vorgesehen, insbesondere parallel zur Längserstreckungsrichtung des Ernteabschnitts und/oder derart positioniert, dass die höchste Lichtintensität außerhalb des Förderbands liegt. Dadurch wird erreicht, dass die Vorpuppen dem Licht entgegen streben. Das Austreiben der Larven aus dem Substrat erfolgt in dieser Ausgestaltung nicht durch eine Beleuchtung des Substrats, sondern mit dem genauen Gegenteil, dem Anlocken der Vorpuppen durch

Licht, da die Lichtquelle so angeordnet ist, dass sie die Larvenbrücke und nicht die Fördervorrichtung, insbesondere das Förderband, beleuchtet. Die Vorpuppen streben zum Licht. Dies ist insbesondere dann vorteilhaft, wenn die Larven bereits zu Vorpuppen entwickelt sind, denn erst in diesem Stadium streben sie zum Licht anstatt davor zu flüchten.

Vorzugsweise ist vorgesehen, dass die Fördervorrichtung, insbesondere ein Förderband der Fördervorrichtung, vor Licht der Lichtquelle der Separiervorrichtung geschützt ist. Insbesondere ist es bevorzugt, dass die Fördervorrichtung nicht von der Lichtquelle der Separiervorrichtung beleuchtet wird und/oder vor Licht, insbesondere ausgehend von der Lichtquelle der Separiervorrichtung, geschützt ist und/oder abgedunkelt und/oder in einem abgedunkelten Bereich angeordnet ist. Bevorzugt ist es, dass die Fördervorrichtung weniger Licht empfangen und/oder empfangen können als die Larvenbrücke. Dies kann vorzugsweise durch Abschirmen des Ernteabschnittes geschehen und/oder durch eine Lenkung des Lichts, mit der Folge, dass so wenig Licht wie möglich auf das Förderband scheint. Vorzugsweise besitzt die Beleuchtung Optiken, die das Licht gezielt richten können.

Insbesondere ist bevorzugt, dass die Ernterampe ein geringes Gefälle in Richtung des Förderbandes aufweist, so dass die Vorpuppen auf dem Weg zum Licht eine Ernterampe aufsteigen müssen, also "bergauf" wandern müssen.

Es wurde erkannt, dass die Vorpuppen scharfe Blechkanten meiden. Daher ist es bevorzugt, dass die Larvenbrücke, die zur Abschöpfung der Vorpuppen eingesetzt wird, verrundete Kanten aufweist. Insbesondere hat sich gezeigt, dass eine Verrundung an der von der Fördervorrichtung, insbesondere dem Förderband, abgewandten Seite besonders vorteilhaft ist, da hierdurch eine Überführung auf ein Vorpuppentransportband auf besonders vorteilhafte Weise gelingt. Die Vorpuppen bewegen sich dabei vorzugsweise von der Fördervorrichtung aus, auf dem sich noch feuchtes Substrat befindet, zum Licht. Auf dem Weg zum Licht müssen sie vorzugsweise über eine Larvenbrücke wandern, die vorteilhafterweise derart auf das Substrat abgesenkt wird, dass sie unmittelbar für die Vorpuppen erreichbar ist. Hierfür besitzt die Larvenbrücke vorzugsweise einen Absenkmechanismus. Wenn die Vorpuppen nun die gesamte Strecke der Larvenbrücke, die aus Sicht der Wegstrecke der Vorpuppe quer zur Längserstreckungsrichtung der Fördervorrichtung verläuft, entlang gewandert ist, treffen sie vorzugsweise auf eine Blechkante die verrundet wurde, wie bspw. durch Abkanten oder Bördeln. Eine scharfkantige Blechkante schreckt die Vorpuppen ab und sie werden nicht animiert über die Kante hinaus nach einem Weg zu suchen. Eine Verrundung kann die Vorpuppen zur Erkundung des weiteren Wegs animieren. Bei dieser Erkundung wagen sie sich vorzugsweise immer weiter über die Verrundung hinaus, bis sie das Gleichgewicht nicht mehr halten können und über die verrundete Blechkante dann in die Vorpuppensammelvorrichtung und/oder auf das Vorpuppentransportband fallen, das vorzugsweise als Endlosförderer ausgebildet ist. Die Verrundung bzw. Abkantung ist vorzugsweise derart gestaltet, dass der Schenkel, welcher sich von der Abkantung in Richtung Boden erstreckt, bei der Position der Abschöpfung im Wesentlichen senkrecht ausgebildet ist. Vorzugsweise ist der Winkel der Abkantung derart gewählt, dass der abgekantete Schenkel des Blechteils in Richtung Boden zeigt und über die Lotrechte hinaus gekantet ist, so dass die Vorpuppen keinen Halt finden können und die Verrundung, an der die Larven auf das Sammelband fallen, wie ein Vorsprung zu verstehen ist. Weiter bevorzugt ist der Radius der Abkantung größer gleich 1,5mm. Noch weiter bevorzugt ist die Abkantung eine Bördelung mit einem Radius größer gleich 1,5mm. In einer weiter bevorzugten Form sieht die Bördelung das vollständige Umschlagen des Bleches um 180° vor.

Vorzugsweise weist die Larvenbrücke eine bestimmte Mindestlänge auf, vorzugsweise größer als 250mm, wodurch sich die Vorpuppen trocken laufen und sich von anhaftenden Substratresten befreien. Durch das bevorzugte Vorsehen der Larvenbrücke entlang des ganzen Larvenseparierabschnitts ist ausreichend Platz für alle Vorpuppen vorhanden, so dass es zu keinem Stau der wandernden Vorpuppen kommt. Damit kann erreicht werden, dass der Wandertrieb der Vorpuppen aufrecht erhalten wird. Ein möglicher Vorpuppenstau kann sich nämlich nachteilig auf den Wandertrieb auswirken.

Es wurde ferner erkannt, dass die Larven durch ihre Bewegungen das Substrat über die gesamte Bandbreite verteilen können, und so das Substrat ebenso auf fest montierten Larvenbrücken tragen würden. Bei einer festen Montage wäre die Rampe in ständigem Kontakt mit dem Substrat und würde dadurch verschmutzt. Weiterhin ergäbe sich an der Stirnseite der Rampe, die der Förderrichtung zugewandt ist, die Möglichkeit der Substratstauung. Vorzugsweise wird daher durch das Herausschwenken und/oder Herausklappen der Larvenbrücken aus dem Substrat ein saubererer Wanderweg gewährleistet, insbesondere wenn die Larvenbrücken nur zur Larvenernte abgesenkt werden, bei der vorzugsweise die Fördervorrichtung stillsteht. Das Absenken der Larvenbrücken kann vorzugsweise manuell oder automatisch geschehen. Insbesondere ist es bevorzugt, dass die Larvenrampe in leichter Überdeckung zum Substrat aufgelegt wird.

Das Abschöpfen der Vorpuppen geschieht vorzugsweise in einem dafür vorgesehenen Abschnitt, insbesondere dem Larvenseparierabschnitt. Um sicherzustellen, dass die Vorpuppen ausreichend Zeit bekommen und sie bei ihrer Wanderung nicht unnötig zu stören, wird die Fördervorrichtung mit dem Substrat für die Zeit der Abschöpfung vollständig angehalten. In einem weiteren Schritt werden die absenkbaren Larvenbrücken auf das Substrat abgesenkt, so dass die Vorpuppen ohne große Mühe auf die Larvenbrücke aufsteigen können und sich in Richtung Lichtquelle bewegen können. Nach Abschöpfung werde die Larvenbrücken wieder angestellt, so dass kein Substrat an den Larvenbrücken haften bleiben kann, wenn die Fördervorrichtung wieder einschaltet wird.

Eine mechanische Trennvorrichtung kann beispielsweise als Sieb und/oder Auswaschvorrichtung und/oder Vibrationsvorrichtung ausgebildet sein.

Vorzugsweise ist die Separiervorrichtung, insbesondere die Larvenbrücke und/oder der Larvenauffangbehälter, mit einem Larvenlockmittel, beispielsweise mit einem für Larven attraktiven Nährstoff und/oder Geschmacksstoff und/oder Duftstoff, versehen.

Vorzugsweise umfasst die Vorrichtung zwei Separiervorrichtungen, wobei die beiden Separiervorrichtungen vorzugsweise unterschiedlich ausgebildet sind und/oder einander nachgeschaltet sind.

Die erste Separiervorrichtung umfasst vorzugsweise eine Lichtquelle, eine Larvenbrücke und einen Larvenauffangbehälter sowie ggf. einen Restkotauffangbehälter. Die zweite, nachgeschaltete Separiervorrichtung umfasst vorzugsweise eine mechanische Trennvorrichtung, wie beispielsweise ein Sieb oder eine Auswaschvorrichtung, und einen Larvenauffangbehälter sowie ggf. einen Restkotauffangbehälter.

Ferner ist es bevorzugt, dass die Förderstrecke, insbesondere der Larveneinbringabschnitt einen Flüssigkeitseinbringabschnitt und/oder einen Durchmischungsabschnitt aufweist.

Als Flüssigkeit ist vorzugsweise Wasser vorgesehen. Die Flüssigkeit wird vorzugsweise aus einem Flüssigkeitsreservoir über einen Vernebler und/oder Sprühkopf und/oder Tropfschlauch und/oder Düsenrohr in Form von Tropfen oder Sprühnebel abgegeben, wodurch vorzugsweise eine gleichmäßige Befeuchtung erzielt wird.

Es wurde erkannt, dass Vorpuppen ebenfalls einen Sinn für Feuchtigkeit bzw. Substratfeuchte besitzen. Im Stadium der Vorpuppe meiden die Larven feuchtes Substrat (bspw. >25% Feuchtigkeit), denn sie streben in trockene Areale, damit sie nach der Verpuppung nicht durch Pilze zerstört werden. Dieser Effekt kann zusätzlich genutzt werden, um die Tier aus dem Substrat zu treiben und zwar in dem zusätzlich zur Beleuchtung der Ernteabschnitt nachbefeuchtet wird.

Der Durchmischungsabschnitt kann beispielsweise als Zwischenförderer mit einer hohen Fördergeschwindigkeit, insbesondere einer Fördergeschwindigkeit, die höher ist als die der Förderstrecke, ausgebildet sein. Der Durchmischungsabschnitt dient insbesondere dazu, eine Umsetzung und/oder Durchmischung des mit Larven und/oder Larveneiern versetzten Tierkots zu erzielen.

Vorzugsweise umfasst die Vorrichtung eine Steuerungsvorrichtung, die ausgebildet ist, zu bestimmen: eine zuzudosierende Menge an Larven und/oder Larveneiern in Abhängigkeit von einem oder mehreren Prozessparametern, und/oder eine zuzudosierende Menge an Tierkot in Abhängigkeit von einem oder mehreren Prozessparametern, und/oder eine zuzudosierende Menge an Flüssigkeit in Abhängigkeit von einem oder mehreren Prozessparametern und/oder eine zuzudosierende Menge an weitere Ressourcen, wie beispielsweise Nährstoffe für die Larven und/oder Larveneier, in Abhängigkeit von einem oder mehreren Prozessparametern.

Prozessparameter können insbesondere sein Menge und Eigenschaften, wie beispielsweise Trockensubstanz (TS)-Gehalt o.ä., von Kot in verschiedenen Prozessphasen (insbesondere des Ausgangskots, des von den Larven verbrauchten Kots, des Restkots), Umgebungsparameter (wie beispielsweise Temperatur und/oder Luftfeuchtigkeit), Länge der Förderstrecke, Durchsatz der Vorrichtung, o.ä. Vorzugsweise umfasse die Vorrichtung einen oder mehrere Sensoren zur Ermittlung von einem oder mehreren Prozessparametern. Die Steuerungsvorrichtung ist vorzugsweise ausgebildet, Prozessparameterdaten zu empfangen, vorzugsweise von einem oder mehreren Sensoren.

Ferner vorzugsweise ist eine Larvendosiervorrichtung vorgesehen, die ausgebildet ist, eine bestimmte Menge an Larven und/oder Larveneiern zuzudosieren in Abhängigkeit von einem oder mehreren Prozessparametern und/oder in Abhängigkeit von einer von der Steuerungsvorrichtung bestimmten zuzudosierenden Menge an Larven.

Vorzugsweise ist das Zudosieren der Junglarven manuell oder automatisiert möglich. Dabei ist vorzugsweise eine Bevorratung einer gewissen Menge an Jungarven mittels eines Bevorratungsbehälters vorgesehen, der bevorzugt als Trichter ausgeführt ist. Am Boden des Bevorratungsbehälters befindet sich vorzugsweise eine Rühreinrichtung, die die Junglarven einem Austrittspunkt zuführt und Verstopfungen verhindert. Diese Rühreinrichtung ist vorzugsweise derart ausgeführt, dass Junglarven nicht gequetscht werden, aber Verklumpung verhindert werden. Erreicht wird dies durch die vorzugsweise Verwendung eines oder mehrerer Rührfinger, bevorzugt Rührflügel aus flexiblem Material. Ferner sind angewinkelte Rührflügel zur schneckenähnlichen Aufrührbewegung bevorzugt, da bei senkrechten Flügeln das Risiko einer Verklumpung und Quetschung der Junglarven besteht.

Die Rührgeschwindigkeit kann vorzugsweise variabel gestaltet sein und ermöglicht vorzugsweise eine Anpassung an unterschiedliche Larvenchargen (bzgl. Feuchte und damit Verklumpungsneigung) sowie an den Bandfortschritt der Fördervorrichtung. Die Austrittsöffnung ist vorzugsweise manuell oder automatisiert in der Größe verstellbar, um auch hiermit eine Quetschung der Larven zu verhindern. Die Austrittsöffnung kann auch ganz verschließbar ausgebildet sein. Eine Mehrfachanordnung der Austrittsöffnungen kann ferner bevorzugt sein, da dadurch eine homogene, gleichmäßigere Verteilung der Larven auf dem Substrat erreicht werden kann.

Unterhalb der Austrittsöffnung befindet sich vorzugsweise eine Larvenverteileinrichtung, die dazu dient, die Larven gleichmäßig auf das Substrat aufzubringen. Dies kann bspw. als rotierender Teller mit aufgeschraubten Winkeleisen ausgeführt sein, vorzugsweise in einer radialsymmetrischen Anordnung zur radialsymmetrischen Verteilung. Denkbar ist auch, dass die Winkeleisen relativ zur Streuachse verstellbar, in oder gegen die Streurichtung, zur Einstellung des Streubildes sind. Eine weitere bevorzugte Ausführung sieht eine variable Einstellung der Rotationsgeschwindigkeit des Tellers zur Einstellung des Streuweite vor, entsprechend der Bandbreite. Ein Betrieb mit variablen Bandbreiten wird somit ermöglicht. Generell ist dieses Ausbringungsprinzip von Düngerstreuern bekannt.

In einer weiteren bevorzugten Ausführungsform ist eine Tierkotdosiervorrichtung vorgesehen, die ausgebildet ist, eine bestimmte Menge an Tierkot zuzudosieren in Abhängigkeit von einem oder mehreren Prozessparametern und/oder in Abhängigkeit von einer von der Steuerungsvorrichtung bestimmten zuzudosierenden Menge an Tierkot.

Vorzugsweise ist eine Verteil- und/oder Zerkleinerungsvorrichtung für Tierkot, der hier auch als Substrat bezeichnet wird, vorgesehen. Die Verteilung und Zerkleinerung des Substrats erfolgt vorzugsweise mittels angetriebener Schnecken. Vorzugsweise ermöglichen sich anschließende Schieber und eine Geschwindigkeitssteuerung der Fördervorrichtung unterschiedliche Substrathöhen.

Ferner vorzugsweise umfasst die Vorrichtung eine Flüssigkeitsdosiervorrichtung, die ausgebildet ist, eine bestimmte Menge an Flüssigkeit zuzudosieren in Abhängigkeit von einem oder mehreren Prozessparametern und/oder in Abhängigkeit von einer von der Steuerungsvorrichtung bestimmten zuzudosierenden Menge an Flüssigkeit.

Eine bevorzugte Weiterbildung zeichnet sich dadurch aus, dass mindestens ein Bereich der Förderstrecke in einer Kottrocknungsvorrichtung angeordnet ist. Ferner ist es bevorzugt, dass verschiedene Bereiche der Förderstrecke in verschiedenen Kottrocknungsvorrichtungen angeordnet sind.

Vorzugsweise sind der Koteinbringabschnitt und der Durchmischungsabschnitt als ein einzelner Abschnitt der Förderstrecke ausgebildet. Der Koteinbringabschnitt und der Durchmischungsabschnitt können als ein und derselbe Abschnitt ausgebildet sein, wobei dieser eine Abschnitt dann beide Funktionen erfüllt, insbesondere sowohl als Koteinbringabschnitt als auch als Durchmischungsabschnitt dient.

Die Vorrichtung zur Reststoff-Verwertung in der Nutztierhaltung ist vorzugsweise in einem klimagesteuerten Raum angeordnet. Vorzugsweise weist die Vorrichtung zur Reststoff-Verwertung in der Nutztierhaltung eine Umhausung auf, beispielsweise einen 40-Fuß Container, insbesondere auch als High Cube Variante, welche den klimagesteuerten Raum bildet. Vorzugsweise ist die Vorrichtung zur Reststoff-Verwertung in der Nutztierhaltung ganz oder teilweise, insbesondere in einem Transportzustand, in der Umhausung angeordnet. Hierdurch wird auch ein einfacher Transport der Vorrichtung zur Reststoff-Verwertung in der Nutztierhaltung möglich. Der klimagesteuerte Raum ermöglicht eine Unabhängigkeit zu den klimatischen Umgebungsparametern wie bspw. Temperatur und Luftfeuchtigkeit. Vorzugsweise ist der klimagesteuerte Raum entsprechend isoliert und/oder klimatisch konditionierbar mit einer Vorrichtung zur Regelung des Klimas als auch mit Aggregaten zur Klimatisierung, d.h. bspw. Kühlung/Erwärmung, Lüftung, Entfeuchtung/Befeuchtung. Ferner ist bevorzugt, dass einer der Klimaparameter in Abhängigkeit der Substrattemperatur geregelt wird, so dass ideale Wachstumsbedingungen für die Larven eingestellt werden können. Ferner bevorzugt ist die Messung des Ammoniakgehaltes der Luft und eine entsprechend davon abhängige Lüftungssteuerung durch Ablüften der Ammoniakgase.

Vorzugsweise weist die Vorrichtung zur Reststoff-Verwertung in der Nutztierhaltung mehrere Ebenen auf, wodurch eine Umsetzung und eine Durchmischung erfolgen kann sowie eine Belüftung des Substrats.

Beispielhafte Ausführung: Die erste Ebene ist bedingt durch die Tierkotdosiervorrichtung halb so lang wie übrige Ebenen, wodurch sich eine höhere Substratmenge im Verhältnis zur Bandlänge der Ebene ergibt. Durch die höhere Substratmenge und sich dadurch ergebende anaerobe Bereiche stellt sich eine leichte Erwärmung ein, die ideal für Junglarven ist, deren metabolische Energie noch nicht ausreicht, das Substrat zu erwärmen. Die zweite bis vierte Ebene ist in etwa doppelt so lang wie erste Ebene, wodurch dem Larvenwachstum Rechnung getragen wird, denn durch das rapide Wachstum ergibt sich ein erhöhter Platzbedarf der wachsenden Larven (40-faches Längenwachstum).

Beispiel Prozessführung:
- 1. (vorzugsweise oberste) Ebene: Junglaven im Alter von vier bis sieben Tagen
- 2.Ebene: mehr Platz für Larven im Alter von sieben bis zehn Tagen
- 3.Ebene: Larven im Alter von zehn bis 13 Tagen
- 4. (vorzugsweise unterste) Ebene: Larven im Alter von 13 bis 15 Tagen und Entwicklung zur Vorpuppe, sowie deren Abwanderung
- Tagesablauf entsprechend der Entwicklung der Larven in unterschiedlichem Substrat anzupassen ist bevorzugt
- Unterschiedliche Geschwindigkeiten der Bänder mit Einrichtungen wie bspw. Frequenzumrichter möglich, um unterschiedliche Substrathöhen und/oder längere Verweildauer in unterster Etage (Ernteetage) zu ermöglichen. Vorzugsweise befinden sich die Vorpuppen nur in der untersten Ebene.

Gemäß einem weiteren Aspekt der Erfindung wird die eingangs genannte Aufgabe gelöst durch eine Anlage zur Reststoff-Verwertung in der Nutztierhaltung, umfassend eine zuvor beschriebene Vorrichtung zur Reststoff-Verwertung in der Nutztierhaltung, eine oder mehrere erste Kottrocknungsvorrichtungen, und eine zweite Kottrocknungsvorrichtung, wobei Bereiche der Förderstrecke der Vorrichtung zur Reststoff-Verwertung als Trocknungsbänder der einen oder mehreren ersten Kottrocknungsvorrichtungen und der zweiten Kottrocknungsvorrichtung ausgebildet sind, wobei der Larveneinbringabschnitt in der ersten Kottrocknungsvorrichtung angeordnet ist, wobei der Koteinbringabschnitt zwischen der ersten und der zweiten Kottrocknungsvorrichtung und/oder zwischen mehreren ersten Kottrocknungsvorrichtungen angeordnet ist, und wobei der Larvenseparierabschnitt in der zweiten Kottrocknungsvorrichtung angeordnet und/oder der zweiten Kottrocknungsvorrichtung nachgeschaltet ist.

Eine bevorzugte Ausgestaltung der Anlage zur Reststoff-Verwertung in der Nutztierhaltung sieht vor, dass der Durchmischungsabschnitt zwischen der ersten und der zweiten Kottrocknungsvorrichtung und/oder zwischen mehreren ersten Kottrocknungsvorrichtungen angeordnet ist.

Gemäß einem weiteren Aspekt der Erfindung wird die eingangs genannte Aufgabe gelöst durch ein Verfahren zur Reststoff-Verwertung in der Nutztierhaltung, vorzugsweise mittels einer zuvor beschriebenen Vorrichtung zur Reststoff-Verwertung in der Nutztierhaltung, das Verfahren umfassend: Fördern von Tierkot entlang einer Förderstrecke, wobei die Förderstrecke einen Larveneinbringabschnitt, einen Koteinbringabschnitt und einen Larvenseparierabschnitt aufweist, Zudosieren von Larven und/oder Larveneiern in dem Larveneinbringabschnitt, Zudosieren von Tierkot in dem Koteinbringabschnitt in Abhängigkeit von einem oder mehreren Prozessparametern, und Separieren der Larven in dem Larvenseparierabschnitt, gekennzeichnet durch Beleuchten einer Larvenbrücke mittels einer Lichtquelle.

Diese weiteren Aspekte und ihre möglichen Fortbildungen weisen Merkmale bzw. Verfahrensschritte auf, die sie insbesondere dafür geeignet machen, für eine bzw. mit einer Vorrichtung zur Reststoff-Verwertung in der Nutztierhaltung und ihren Fortbildungen verwendet zu werden.

Zu den Vorteilen, Ausführungsvarianten und Ausführungsdetails dieser weiteren Aspekte der Erfindung und ihrer Fortbildungen wird auf die vorangegangene Beschreibung zu den entsprechenden Vorrichtungsmerkmalen verwiesen.

Bevorzugte Ausführungsformen der Erfindung werden beispielhaft anhand der beiliegenden Figuren beschrieben. Es zeigen:
- Figur 1:: eine schematische Darstellung eines Beispiels einer Vorrichtung zur Reststoff-Verwertung in der Nutztierhaltung;
- Figur 2:: ein schematisches Ablaufdiagramm eines Beispiels für ein Verfahren zur Reststoff-Verwertung in der Nutztierhaltung mit einer Vorrichtung zur Reststoff-Verwertung in der Nutztierhaltung;
- Figur 3:: eine dreidimensionale Ansicht einer Kottrocknungsvorrichtung;
- Figur 4:: eine dreidimensionale Ansicht eines Teils einer beispielhaften Vorrichtung zur Reststoff-Verwertung in der Nutztierhaltung;
- Figur 5:: eine Frontansicht der Vorrichtung gemäß Figur 2;
- Figur 6:: eine Detaildarstellung der gezeigten Separiervorrichtung;
- Figur 7:: eine Detaildarstellung der Lichtquelle der in Figur gezeigten Separiervorrichtung;
- Figur 8:: eine schematische Darstellung eines Teils einer beispielhaften Vorrichtung zur Reststoff-Verwertung in der Nutztierhaltung mit Separiervorrichtung;
- Figur 9:: eine schematische Darstellung einer beispielhaften Vorrichtung zur Reststoff-Verwertung in der Nutztierhaltung;
- Figur 10:: einen vergrößerten Ausschnitt aus Figur 9; und
- Figur 11:: einen weiteren vergrößerten Ausschnitt aus Figur 9.

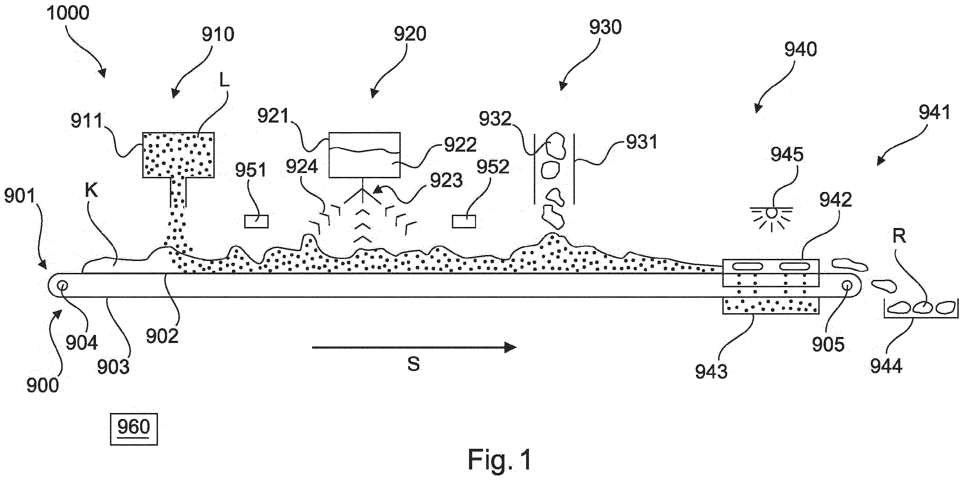

In Figur 1 ist ein Beispiel einer Vorrichtung 1000 zur Reststoff-Verwertung in der Nutztierhaltung beispielhaft und schematisch dargestellt.

Figur 1 zeigt eine Vorrichtung 1000 zur Reststoff-Verwertung in der Nutztierhaltung mit einer Fördervorrichtung 900 zum Fördern von Tierkot K entlang einer Förderstrecke S. die Fördervorrichtung 900 ist als Bandförderer mit einem Endlosband 901 mit einem Obertrum 902, auf dem der Kot K gefördert wird, und einem Untertrum 903, die über Umlenkrollen 904, 905 umgelenkt werden, ausgebildet. Eine der Umlenkrollen 904, 905 ist vorzugsweise elektrisch angetrieben.

Die Förderstrecke S weist einen Larveneinbringabschnitt 910 zum Zudosieren von Larven und/oder Larveneiern L, einen Flüssigkeitseinbringabschnitt 920 zum Zudosieren von Flüssigkeit 922 einen Koteinbringabschnitt 930 zum Zudosieren von Tierkot K in Abhängigkeit von einem oder mehreren Prozessparametern und einen Larvenseparierabschnitt 940 zum Separieren der Larven L auf. Die Larven und/oder Larveneiern L können von einem Larvenreservoir 911 aus zudosiert werden. Die Flüssigkeit 922, insbesondere Wasser, kann aus einem Flüssigkeitsreservoir 921 über einen Vernebler oder Sprühkopf 923 in Form von Tropfen oder Sprühnebel 924 abgegeben werden. Der zuzudosierende Kot 932 kann über eine Kotzufuhreinrichtung 931, vorzugweise ebenfalls ein Bandförderer, im Koteinbringabschnitt 930 zugeführt werden.

Vorzugsweise sind in der Vorrichtung 1000 ein oder mehrere Sensoren 951, 952 vorgesehen, die einen oder mehrere Prozessparameter erfassen. Von einer Steuerungsvorrichtung 960 können vorzugsweise aus diesen Prozessparametern zuzudosierende Mengen an Larven und/oder Larveneiern L und/oder Flüssigkeit 922 und/oder zuzudosierendem Kot 932 bestimmt werden.

Im Larvenseparierabschnitt 940 ist eine Separiervorrichtung 941 mit einem Larvenauffangbehälter 943 und einem Restkotauffangbehälter 944 angeordnet. Die Separiervorrichtung 941 weist ferner eine Lichtquelle 945 auf. Vom Larvenseparierabschnitt 940 führt eine Larvenbrücke 942 zum Larvenauffangbehälter 943. Ein Inneres des

Larvenauffangbehälters 943 ist vorzugsweise vor Licht der Lichtquelle 945 geschützt. Auf diese Weise werden die Larven L im Larvenseparierabschnitt 940 angeregt, den beleuchteten Bereich zu verlassen und über die Larvenbrücke 942 in den Larvenbehälter 943 zu gelangen, vorzugsweise durch Eigenbewegung. Auf diese Weise kann eine besonders einfache Trennung der Larven L vom Restkot R erfolgen.

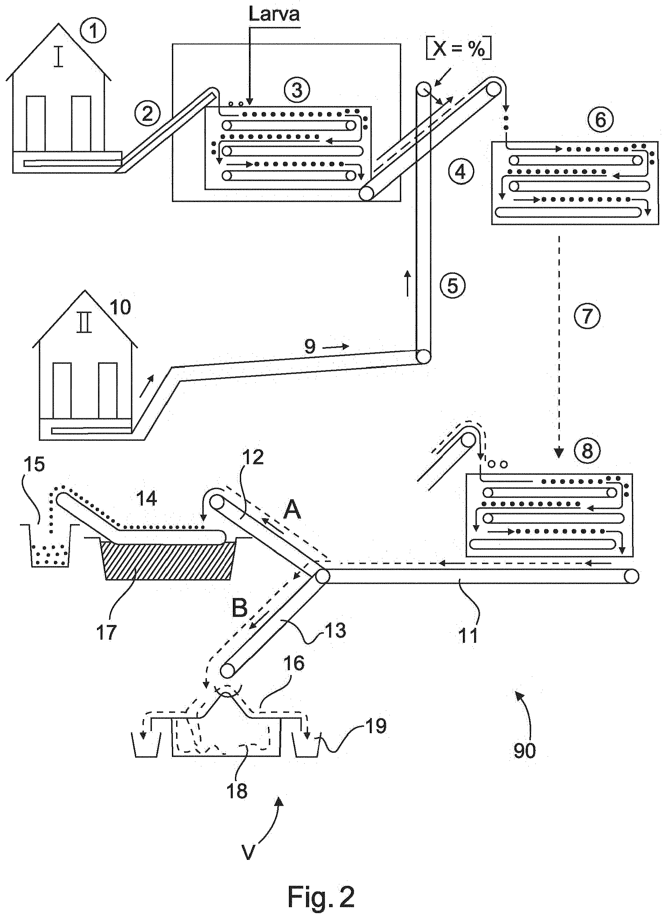

Figur 2 zeigt ein schematisches Ablaufdiagramm eines Beispiels für ein Verfahren V zur Reststoff-Verwertung in der Nutztierhaltung mit einer Vorrichtung 90 zur Reststoff-Verwertung in der Nutztierhaltung.

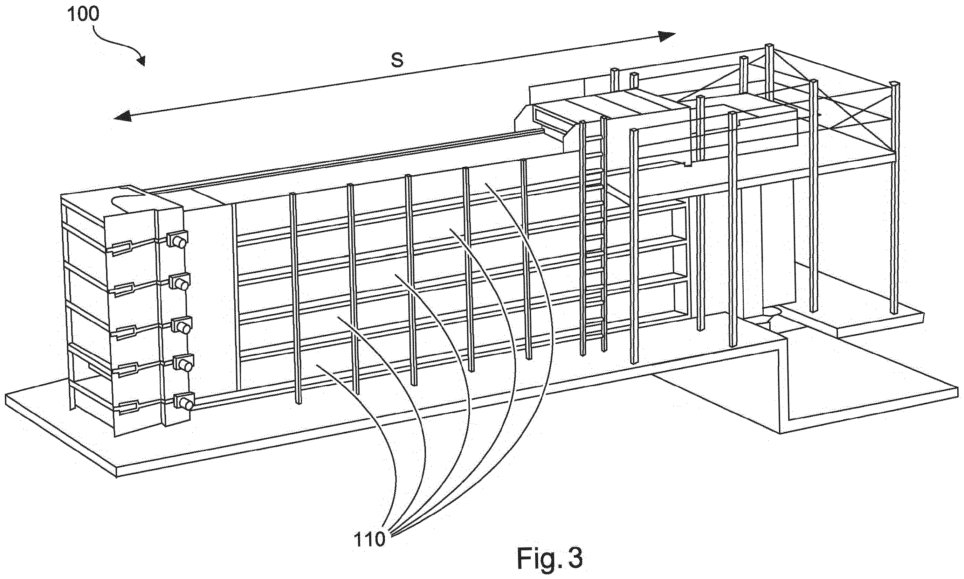

Figur 3 zeigt eine dreidimensionale Ansicht einer Kottrocknungsvorrichtung 100 mit fünf Etagen 110. Die in Figur 3 nur schematisch angedeutete Förderstrecke S verläuft über gegenläufige, angetriebene Fördervorrichtungen in den übereinanderliegenden Etagen. Die Etagenanordnung bietet auch den Vorteil, dass bei der Übergabe des Kot-Larvengemisches zwischen den Etagen eine Umwälzung durch Herabfallen auf die untere Etage stattfindet und somit den Larven verwertbares (Kot-)Material freilegt. Mehrere solcher Kottrocknungsvorrichtungen 100 können in Reihe positioniert werden und zusammen eine Förderstrecke bilden für eine Vorrichtung zur Reststoff-Verwertung in der Nutztierhaltung. Die Anzahl der erforderlichen Kottrocknungsvorrichtungen 100 variiert je nach Menge des zu trocknenden Tierkots und/oder der zu kultivierenden Larven.

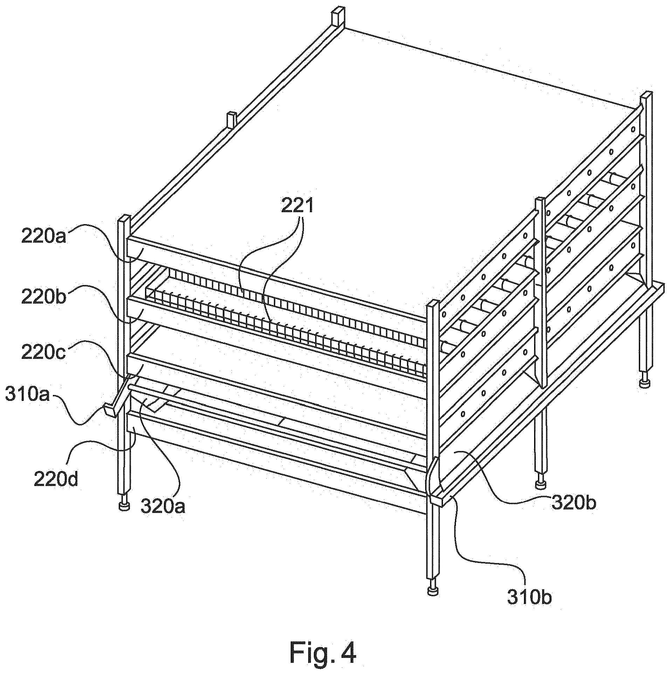

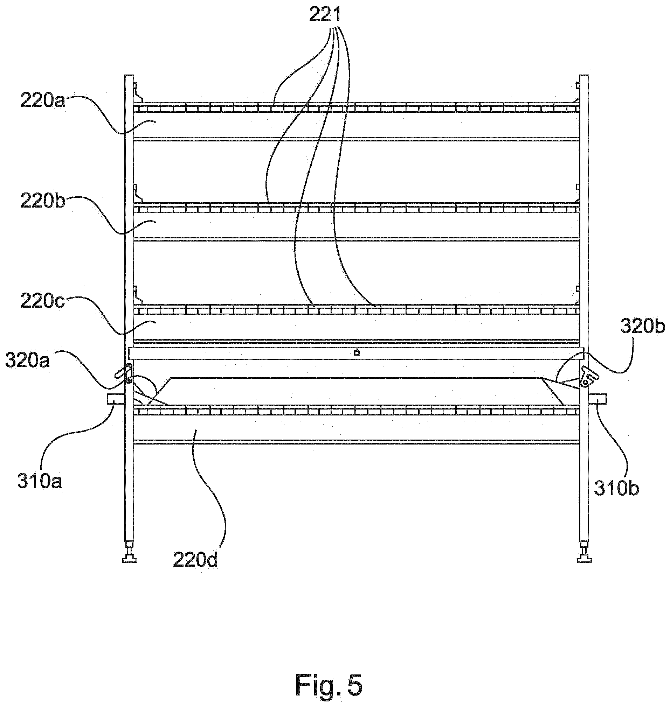

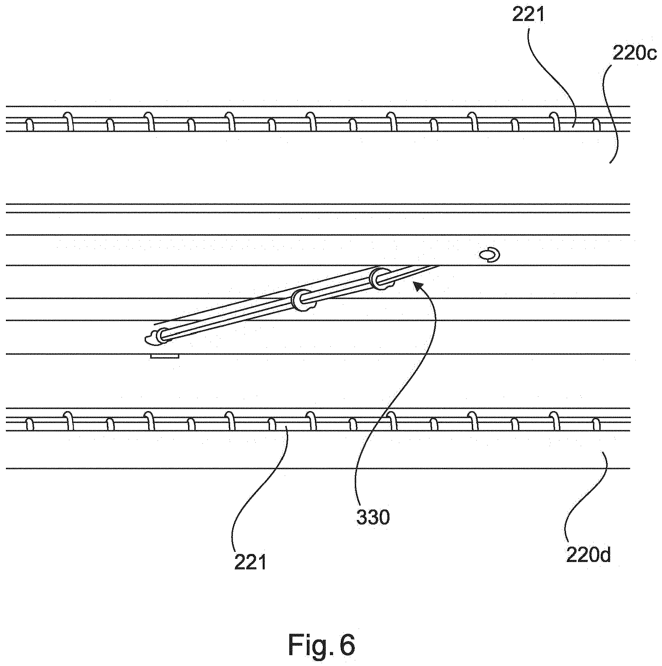

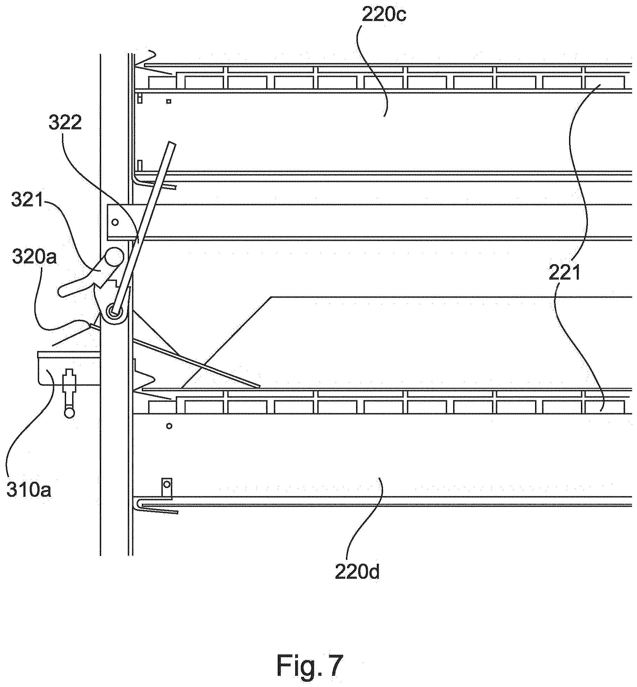

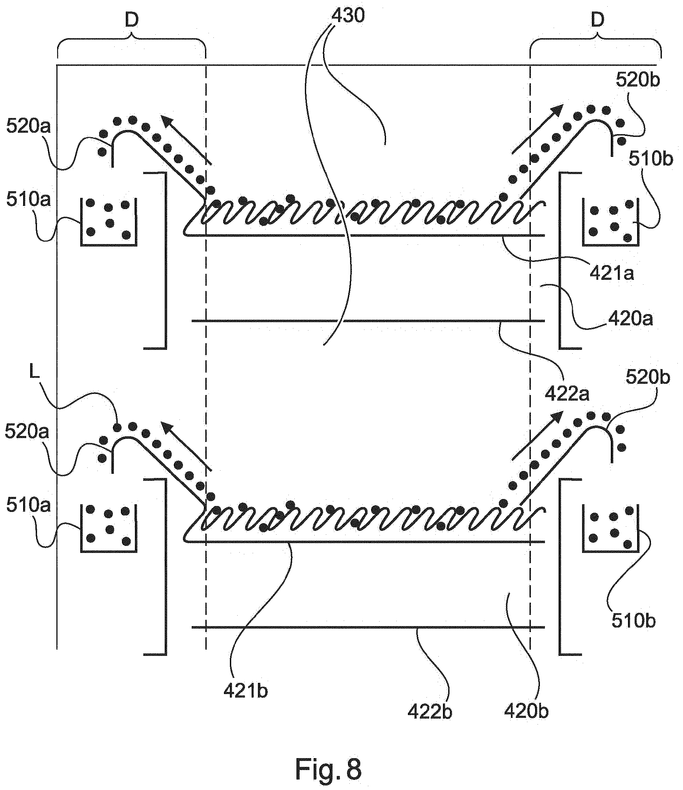

In den Figuren 4 bis 7 und 8 sind Möglichkeiten von Separiervorrichtungen näher dargestellt. Die Förderstrecke erstreckt sich in den in den Figuren 4 bis 7 und 8 gezeigten Bereichen über mehrere Ebenen 220a,b,c,d, 420a,b. In Figur 8 sind in den beiden Ebenen 420a,b jeweils der Obertrum 421a,b und der Untertrum 422a,b, der Bandförderer zu erkennen. In den Figuren 4 bis 7 sind die Unterzüge 221 der Bandförderer zu erkennen.

Beim Ernten der Larven in der letzten Kottrocknungsvorrichtung kann für die Bewegungskontrolle der Larven eine Separiervorrichtung mit einer Lichtquelle 330, 430 verwendet werden. Grundsätzlich wird mit diesem Ansatz die Lichtsensitivität der Larven ausgenutzt.

Zum Ernten der Larven sind an den Seiten der Förderstrecke im Abstand D Larvenauffangbehälter 310a,b, 510a,b angeordnet. Die Larven L können über als Bleche ausgebildete Larvenbrücken 320a,b, 520a,b in die Larvenauffangbehälter 310a,b, 510a,b gelangen, vorzugsweise durch Eigenbewegung, die durch die Lichtquelle 330, 430 verursacht oder unterstützt wird. Die Larvenbrücken können beweglich ausgestaltet sein von einer Position 320a, in der die Larven über die Larvenbrücken 320a, in die Larvenauffangbehälter 310a gelangen können, in eine Position 320b, in der dies nicht gegeben ist, und zurück. So können beispielsweise Beginn und Ende der Larvenernte gesteuert werden, vorzugsweise in Abstimmung mit dem Betrieb der Lichtquelle. Vorzugsweise bleibt in der Position 320b die Fördervorrichtung, insbesondere das Kotband, auf dem der Kot sowie die Larven aufliegen, noch beweglich und kann somit angetrieben werden. Hierzu ist die Larvenbrücke in der Position 320b vorzugsweise hochgekappt. Vorzugsweise ist ein Absenkmechanismus vorgesehen, vorzugsweise in Form einer Verstell- und/oder Feststelleinrichtung, um die hier als metallische Seitenplatte ausgebildete Larvenbrücke zur Steuerung der Larvenbewegung in die bzw. in der Position 320a, b zu verstellen und/oder festzustellen. Die Verstell- und/oder Feststelleinrichtung weist vorzugsweise einen Verstellmechanismus 322, beispielsweise einen Hebel, auf. Der Verstellmechanismus 322 ist insbesondere ausgebildet, die Larvenbrücke von der Position 320a in die Position 320b und/oder umgekehrt zu bringen. Ferner vorzugsweise weist die Verstell- und/oder Feststelleinrichtung einen Fixiermechanismus 321, beispielsweise einen Haken auf, der insbesondere ausgebildet ist, die Larvenbrücke in der Position 320b zu fixieren.

Im folgenden wird das in Figur 2 schematisch dargestellte Verfahren V zur Reststoff-Verwertung in der Nutztierhaltung mit einer Vorrichtung 90 zur Reststoff-Verwertung in der Nutztierhaltung näher erläutert. Der aus einem ersten Geflügeltierstall I, 1 stammende Geflügelkot wird über eine Zufördervorrichtung 2 einer ersten Kottrocknungsvorrichtung 3 zugeführt und über die gesamte Breite des Trocknungsbandes verteilt. Über eine kontinuierliche Zuführung wird so schlussendlich die gesamte Förderstrecke mit Geflügelkot befüllt. Die erste Kottrocknungsvorrichtung 3 entspricht im in Figur 2 gezeigten Beispiel dem Larveneinbringabschnitt. Hier werden Larven und/oder Larveneier in einem spezifischen Mischungsverhältnis in den Kot eingebracht.

Der Tierkot inkl. Larven und/oder Larveneiern wird durch das angetriebene Kotband des Bandförderers der Fördervorrichtung durch die erste Kottrocknungsvorrichtung 3 transportiert. Aufgrund der Nahrungsaufnahme durch die Larven reduziert sich die Kotmenge. Sobald die erste Kottrocknungsvorrichtung 3 durchlaufen ist, wird der Tierkot über einen Durchmischungsabschnitt 4 zu einer zweiten Kottrocknungsvorrichtung 6 gefördert. Der Durchmischungsabschnitt 4 ist hier identisch mit einem Koteinbringabschnitt ausgebildet, wo weiterer Tierkot aus einem zweiten Geflügeltierstall II, 10 über Zufördervorrichtungen 9, 5, zugeführt wird. Diese Zudosierung von Tierkot, beispielsweise X% der Ausgangskotmenge, erfolgt, um eine ausreichende Nahrungsverfügbarkeit für die Larven und/oder Larveneiern und somit einen optimalen Wachstum sicherzustellen und vorzugsweise in Abhängigkeit von einem oder mehreren Prozessparametern.

Die Zuführung von Tierkot muss nicht direkt aus einem Nutztierstall erfolgen. Eine Zwischenlagerung des Tierkots bzw. eine dezentrale Lösung sind ebenfalls möglich.

Dieser Prozess mit Durchmischung und Zuführung von neuem Tierkot zwischen zwei Trocknungsvorrichtungen kann sich mehrfach wiederholen. Entscheidend für die Anzahl der Wiederholungen ist u.a. die zu verarbeitende Menge an Tierkot, die Wachstumszeit der Larven und/oder Larveneier sowie die angestrebte Menge der Larvenproduktion. Der Prozess ist an dieser Stelle insbesondere dadurch skalierbar, dass eine variable Anzahl an Kottrocknungsvorrichtungen 7 (in Figur 2 nur schematisch angedeutet) hinzugefügt werden kann.

Die letzte Kottrocknungsvorrichtung 8 unterscheidet sich von den vorgeschalteten Kottrocknungsvorrichtungen insbesondere durch den Larvenseparierabschnitt zum Separieren der Larven, insbesondere durch die Separiervorrichtung. Dies ist insbesondere in den Figuren 3 bis 7 und 8 näher gezeigt und dort näher beschrieben.

Falls nicht sämtliche Larven mittels Lichtsteuerung geerntet werden können, können eine oder mehrere weitere Separiervorrichtungen nachgeschaltet werden. Hierzu kann das Restmaterial aus Restkot und verbliebenen Larven, welches in der letzten Kottrocknungsvorrichtung 8 befindlich ist, zunächst über ein weiteres Fördersystem 11 ausgetragen werden. Ein anschließendes Ernten der im Restkot verbliebenen Larven kann über die folgenden beiden Optionen erfolgen:
A - Ernten Mittels Waschung: Hierbei wird der Restkot mit den verbliebenen Larven über eine Zufördervorrichtung 12 auf eine Auswaschvorrichtung 14 gefördert und dort ausgewaschen. Ausgewaschener Restkot wird, zusammen mit der Waschflüssigkeit oder getrennt davon, in einem Restkotauffangbehälter 17 gesammelt und die gereinigten Larven werden in einem Larvenauffangbehälter 15 aufgefangen.
B - Ernten mittels Siebung: Hierbei wird der Restkot mit den verbliebenen Larven über eine Zufördervorrichtung 13 auf ein Sieb 16 gefördert, wo die Larven abgesiebt und über Schubkästen 18 unterhalb des Siebes 16 aufgefangen werden. Der verbleibende Restkot wird in Restkotauffangbehälter 19 abgetragen.

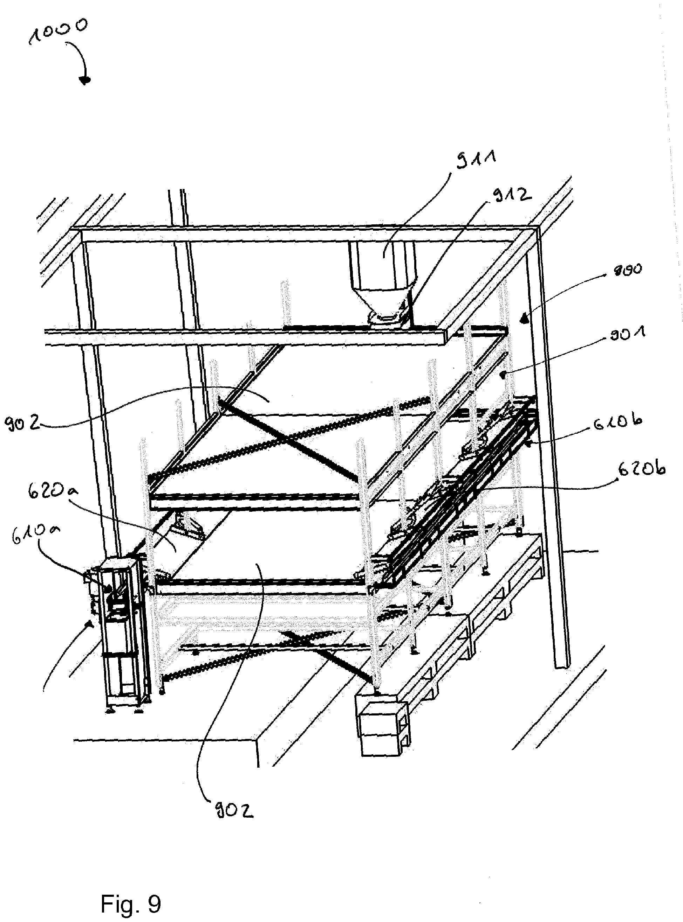

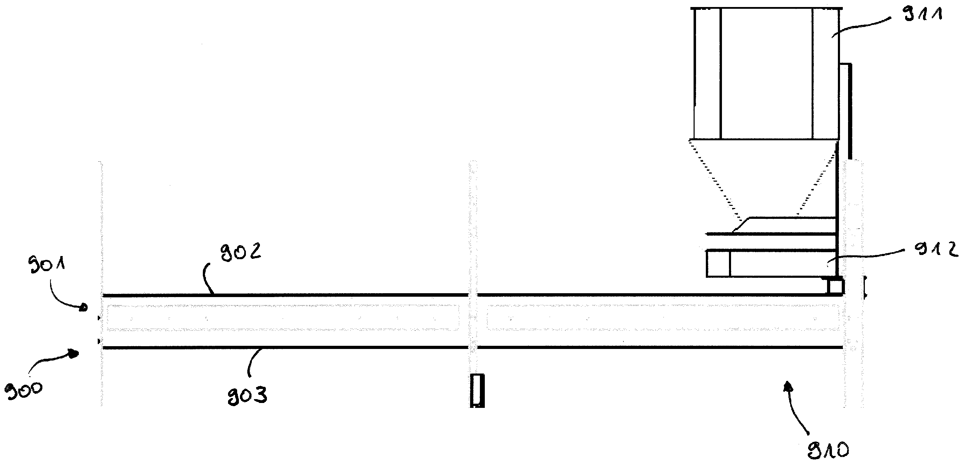

In Figuren 9-11 ist ein weiteres Beispiel einer Vorrichtung 1000 zur Reststoff-Verwertung in der Nutztierhaltung schematisch dargestellt. Die Vorrichtung 1000 zur Reststoff-Verwertung in der Nutztierhaltung weist eine als Bandförderer ausgebildeten Fördervorrichtung 900 mit einem Endlosband 901 mit einem Obertrum 902 und einem Untertrum 903, zum Fördern von Tierkot entlang einer Förderstrecke auf.

Im Larveneinbringabschnitt 910 zum Zudosieren von Junglarven sind ein Larvenreservoir 911 und eine Larvenverteileinrichtung 912 angeordnet.

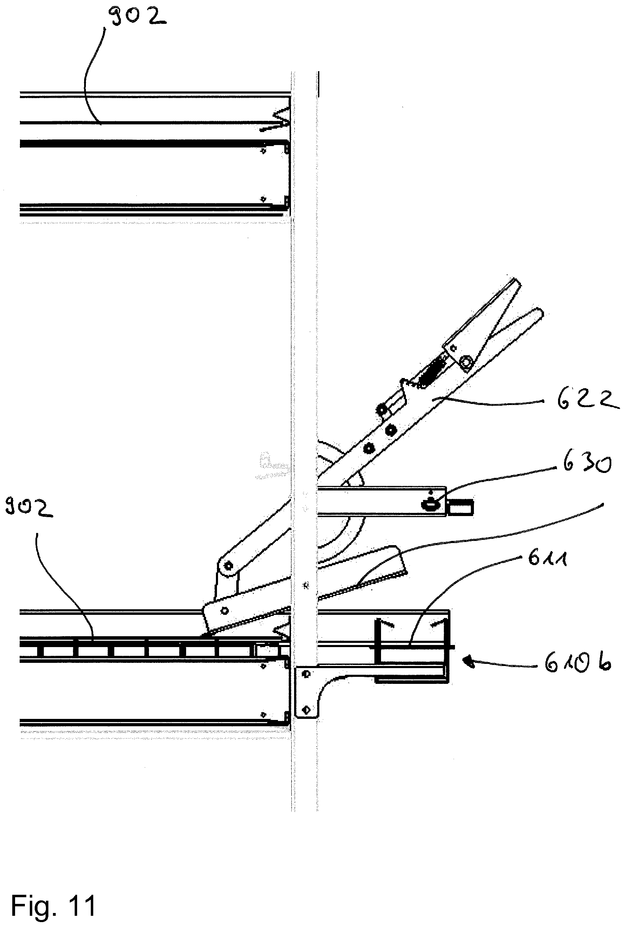

Zum Ernten der Larven im Stadium der Vorpuppen sind an den Seiten der Förderstrecke Larvenauffangbehälter 610a,b angeordnet, hier in Form von U-förmigen Larvenkanälen mit Larventransportbändern 611. Die Vorpuppen können über als Bleche mit verrundeten Kanten ausgebildete Larvenbrücken 620a,b in die Larvenauffangbehälter 610a,b gelangen, vorzugsweise durch Eigenbewegung, die durch die Lichtquelle 630 verursacht oder unterstützt wird. Die Larvenbrücken können mitttels einer als Absenkmechanismus ausgebildeten Verstelleinrichtung 622 beweglich ausgestaltet sein von der in Fig. 11 dargestellten abgesenkten Position, in der die Larven über die Larvenbrücken 620a, in die Larvenauffangbehälter 610a gelangen können, in eine angehobene Position, in der dies nicht gegeben ist, und zurück. So können beispielsweise Beginn und Ende der Larvenernte gesteuert werden, vorzugsweise in Abstimmung mit dem Betrieb der Lichtquelle 630. Die Lichtquelle 630 ist angeordnet, die Larvenbrücke 620a zu beleuchten, nicht jedoch das Obertrum 902 des Förderbands 901.

## Patentansprüche

1. Vorrichtung (1000) zur Reststoff-Verwertung in der Nutztierhaltung, umfassend
- eine Fördervorrichtung (900) zum Fördern von Tierkot entlang einer Förderstrecke (S),
- wobei die Förderstrecke einen Larveneinbringabschnitt (910) zum Zudosieren von Larven und/oder Larveneiern, einen Koteinbringabschnitt (930) zum Zudosieren von Tierkot in Abhängigkeit von einem oder mehreren Prozessparametern und einen Larvenseparierabschnitt (940) zum Separieren der Larven aufweist,
- eine Separiervorrichtung, die eine Lichtquelle (630) und eine Larvenbrücke (620a, 620b) aufweist,
**dadurch gekennzeichnet, dass** die Lichtquelle (630) angeordnet und/oder ausgebildet ist, die Larvenbrücke (620a, 620b) zu beleuchten.

2. Vorrichtung nach dem vorhergehenden Anspruch,
wobei die Separiervorrichtung einen Larvenauffangbehälter aufweist.

3. Vorrichtung nach mindestens einem der vorhergehenden Ansprüche, umfassend einen Restkotauffangbehälter.

4. Vorrichtung nach mindestens einem der vorhergehenden Ansprüche,
wobei die Lichtquelle parallel zur Längserstreckungsrichtung des Larvenseparierabschnitts angeordnet ist.

5. Vorrichtung nach mindestens einem der vorhergehenden Ansprüche,
wobei die Fördervorrichtung, insbesondere ein Förderband der Fördervorrichtung, vor Licht der Lichtquelle der Separiervorrichtung geschützt ist.

6. Vorrichtung nach mindestens einem der vorhergehenden Ansprüche, umfassend zwei Separiervorrichtungen, wobei die beiden Separiervorrichtungen vorzugsweise unterschiedlich ausgebildet sind und/oder einander nachgeschaltet sind.

7. Vorrichtung nach mindestens einem der vorhergehenden Ansprüche,
wobei die Förderstrecke, insbesondere der Larveneinbringabschnitt, einen Flüssigkeitseinbringabschnitt und/oder einen Durchmischungsabschnitt aufweist.

8. Vorrichtung nach mindestens einem der vorhergehenden Ansprüche, umfassend eine Steuerungsvorrichtung, die ausgebildet ist, zu bestimmen
- eine zuzudosierende Menge an Larven und/oder Larveneiern in Abhängigkeit von einem oder mehreren Prozessparametern, und/oder
- eine zuzudosierende Menge an Tierkot in Abhängigkeit von einem oder mehreren Prozessparametern, und/oder
- eine zuzudosierende Menge an Flüssigkeit in Abhängigkeit von einem oder mehreren Prozessparametern.

9. Vorrichtung nach mindestens einem der vorhergehenden Ansprüche,
- umfassend eine Larvendosiervorrichtung, die ausgebildet ist, eine bestimmte Menge an Larven und/oder Larveneiern zuzudosieren in Abhängigkeit von einem oder mehreren Prozessparametern und/oder in Abhängigkeit von einer von der Steuerungsvorrichtung bestimmten zuzudosierenden Menge an Larven und/oder Larveneiern, und/oder
- umfassend eine Tierkotdosiervorrichtung, die ausgebildet ist, eine bestimmte Menge an Tierkot zuzudosieren in Abhängigkeit von einem oder mehreren Prozessparametern und/oder in Abhängigkeit von einer von der Steuerungsvorrichtung bestimmten zuzudosierenden Menge an Tierkot, und/oder
- umfassend eine Flüssigkeitsdosiervorrichtung, die ausgebildet ist, eine bestimmte Menge an Flüssigkeit zuzudosieren in Abhängigkeit von einem oder mehreren Prozessparametern und/oder in Abhängigkeit von einer von der Steuerungsvorrichtung bestimmten zuzudosierenden Menge an Flüssigkeit.

10. Vorrichtung nach mindestens einem der vorhergehenden Ansprüche,
**dadurch gekennzeichnet, dass** mindestens ein Bereich der Förderstrecke in einer Kottrocknungsvorrichtung angeordnet ist; und/oder **dadurch gekennzeichnet, dass** verschiedene Bereiche der Förderstrecke in verschiedenen Kottrocknungsvorrichtungen angeordnet sind.

11. Vorrichtung nach mindestens einem der vorhergehenden Ansprüche,
**dadurch gekennzeichnet, dass** die Förderstrecke zwei oder mehrere Teilförderstrecken übereinander umfasst, die vorzugsweise gegenläufige Förderrichtungen aufweisen und/oder vorzugsweise durch mehrere übereinander angeordnete Bandförderer gebildet sind.

12. Vorrichtung nach mindestens einem der vorhergehenden Ansprüche,
**dadurch gekennzeichnet, dass** der Koteinbringabschnitt und der Durchmischungsabschnitt als ein einzelner Abschnitt der Förderstrecke ausgebildet sind.

13. Anlage zur Reststoff-Verwertung in der Nutztierhaltung,
umfassend eine Vorrichtung zur Reststoff-Verwertung in der Nutztierhaltung nach mindestens einem der vorhergehenden Ansprüche, eine oder mehrere erste Kottrocknungsvorrichtungen, und eine zweite Kottrocknungsvorrichtung,
- wobei Bereiche der Förderstrecke der Vorrichtung zur Reststoff-Verwertung als Trocknungsbänder der einen oder mehreren ersten Kottrocknungsvorrichtungen und der zweiten Kottrocknungsvorrichtung ausgebildet sind,
- wobei der Larveneinbringabschnitt in der ersten Kottrocknungsvorrichtung angeordnet ist,
- wobei der Koteinbringabschnitt zwischen der ersten und der zweiten Kottrocknungsvorrichtung und/oder zwischen mehreren ersten Kottrocknungsvorrichtungen angeordnet ist, und
- wobei der Larvenseparierabschnitt in der zweiten Kottrocknungsvorrichtung angeordnet und/oder der zweiten Kottrocknungsvorrichtung nachgeschaltet ist.

14. Anlage zur Reststoff-Verwertung in der Nutztierhaltung nach dem vorhergehenden Anspruch,
wobei der Durchmischungsabschnitt zwischen der ersten und der zweiten Kottrocknungsvorrichtung und/oder zwischen mehreren ersten Kottrocknungsvorrichtungen angeordnet ist.

15. Verfahren zur Reststoff-Verwertung in der Nutztierhaltung,
umfassend:
- Fördern von Tierkot entlang einer Förderstrecke,
- wobei die Förderstrecke einen Larveneinbringabschnitt, einen Koteinbringabschnitt und einen Larvenseparierabschnitt aufweist,
- wobei der Larvenseparierabschnitt eine Separiervorrichtung mit einer Lichtquelle (630) und einer Larvenbrücke (620a, 620b) aufweist,
- Zudosieren von Larven und/oder Larveneiern in dem Larveneinbringabschnitt,
- Zudosieren von Tierkot in dem Koteinbringabschnitt in Abhängigkeit von einem oder mehreren Prozessparametern,
- Separieren der Larven in dem Larvenseparierabschnitt, **gekennzeichnet durch**
- Beleuchten der Larvenbrücke mittels der Lichtquelle.

## Claims

1. Device (1000) for residue use in livestock farming, comprising
- a conveyor device (900) for conveying animal excrement along a conveyor section (S),
- wherein the conveyor section has a larva-introducing portion (910) for metering in larvae and/or larva eggs, an excrement-introducing portion (930) for metering in animal excrement depending on one or more process parameters, and a larva-separating portion (940) for separating the larvae,
- a separating device comprising a light source (630) and a larva bridge (620a, 620b),
**characterized in that** the light source (630) is arranged and/or designed to illuminate the larva bridge (620a, 620b).

2. Device according to the preceding claim,
wherein the separating device comprises a larva-collecting container.

3. Device according to at least one of the preceding claims,
comprising a residual-excrement-collecting container.

4. Device according to at least one of the preceding claims,
wherein the light source is arranged parallel to the direction of longitudinal extent of the larva-separating portion.

5. Device according to at least one of the preceding claims,
wherein the conveyor device, in particular a conveyor belt of the conveyor device, is protected from light of the light source of the separating device.

6. Device according to at least one of the preceding claims,
comprising two separating devices, wherein the two separating devices are preferably designed differently and/or one is connected downstream of the other.

7. Device according to at least one of the preceding claims,
wherein the conveyor section, in particular the larva-introducing portion, has a liquid-introducing portion and/or a mixing portion.

8. Device according to at least one of the preceding claims,
comprising a control device which is designed to determine
- a quantity of larvae and/or larva eggs to be metered in depending on one or more process parameters, and/or
- a quantity of animal excrement to be metered in depending on one or more process parameters, and/or
- a quantity of liquid to be metered in depending on one or more process parameters.

9. Device according to at least one of the preceding claims,
- comprising a larva-metering device which is designed to meter in a certain quantity of larvae and/or larva eggs depending on one or more process parameters and/or depending on a quantity of larvae and/or larva eggs which is determined by the control device and is to be metered in, and/or
- comprising an animal-excrement-metering device which is designed to meter in a certain quantity of animal excrement depending on one or more process parameters and/or depending on a quantity of animal excrement which is determined by the control device and is to be metered in, and/or
- comprising a liquid-metering device which is designed to meter in a certain quantity of liquid depending on one or more process parameters and/or depending on a quantity of liquid which is determined by the control device and is to be metered in.

10. Device according to at least one of the preceding claims,
**characterized in that** at least one region of the conveyor section is arranged in an excrement-drying device; and/or
**characterized in that** different regions of the conveyor section are arranged in different excrement-drying devices.

11. Device according to at least one of the preceding claims,
**characterized in that** the conveyor section comprises two or more partial conveyor sections one above another, which preferably have opposed conveyor directions and/or are preferably formed by a plurality of belt conveyors arranged one above another.

12. Device according to at least one of the preceding claims,
**characterized in that** the excrement-introducing portion and the mixing portion are designed as an individual portion of the conveyor section.

13. System for residue use in livestock farming,
comprising a device for residue use in livestock farming according to at least one of the preceding claims, one or more first excrement-drying devices, and a second excrement-drying device,
- wherein regions of the conveyor section of the device for residue use are designed as drying belts of the one or more first excrement-drying devices and of the second excrement-drying device,
- wherein the larva-introducing portion is arranged in the first excrement-drying device,
- wherein the excrement-introducing portion is arranged between the first and the second excrement-drying device and/or between a plurality of first excrement-drying devices, and
- wherein the larva-separating portion is arranged in the second excrement-drying device and/or is arranged downstream of the second excrement-drying device.

14. System for residue use in livestock farming according to the preceding claim, wherein the mixing portion is arranged between the first and the second excrement-drying device and/or between a plurality of first excrement-drying devices.

15. Method for residue use in livestock farming,
comprising:
- conveying animal excrement along a conveyor section,
- wherein the conveyor section has a larva-introducing portion, an excrement-introducing portion and a larva-separating portion,
- wherein the larva-separating portion comprises a separating device with a light source (630) and a larva bridge (620a, 620b),
- metering in larvae and/or larva eggs in the larva-introducing portion,
- metering in animal excrement in the excrement-introducing portion depending on one or more process parameters,
- separating the larvae in the larva-separating portion **characterized by**
- illuminating the larva bridge by the light source.

## Revendications

1. Dispositif (1000) de valorisation de résidus dans l'élevage d'animaux de rente, comprenant
- un dispositif de convoyage (900) pour convoyer des excréments d'animaux le long d'une ligne de convoyage (S),
- dans lequel la ligne de convoyage présente un tronçon d'introduction de larves (910) pour ajouter de manière dosée des larves et/ou des œufs de larve, un tronçon d'introduction d'excréments (930) pour ajouter de manière dosée des excréments d'animaux en fonction d'un ou de plusieurs paramètres de processus et un tronçon de séparation de larves (940) pour séparer les larves,
- un dispositif de séparation, qui présente une source de lumière (630) et un pont de larves (620a, 620b),
**caractérisé en ce que** la source de lumière (630) est disposée et/ou réalisée pour éclairer le pont de larves (620a, 620b).

2. Dispositif selon la revendication précédente,
dans lequel le dispositif de séparation présente un contenant de collecte de larves.

3. Dispositif selon au moins l'une quelconque des revendications précédentes, comprenant un contenant de collecte de restes d'excréments.

4. Dispositif selon au moins l'une quelconque des revendications précédentes, dans lequel la source de lumière est disposée de manière parallèle par rapport à la direction d'extension longitudinale du tronçon de séparation de larves.

5. Dispositif selon au moins l'une quelconque des revendications précédentes, dans lequel le dispositif de convoyage, en particulier une bande de convoyage du dispositif de convoyage, est protégé(e) d'une lumière de la source de lumière du dispositif de séparation.

6. Dispositif selon au moins l'une quelconque des revendications précédentes, comprenant deux dispositifs de séparation, dans lequel les deux dispositifs de séparation sont réalisés de préférence différemment et/ou sont installés l'un après l'autre.

7. Dispositif selon au moins l'une quelconque des revendications précédentes,
dans lequel la ligne de convoyage, en particulier le tronçon d'introduction de larves, présente un tronçon d'introduction de liquide et/ou un tronçon de mélange.

8. Dispositif selon au moins l'une quelconque des revendications précédentes,
comprenant un dispositif de commande, qui est réalisé pour définir
- une quantité à ajouter de manière dosée de larves et/ou d'œufs de larves en fonction d'un ou de plusieurs paramètres de processus, et/ou
- une quantité à ajouter de manière dosée d'excréments d'animaux en fonction d'un ou de plusieurs paramètres de processus, et/ou
- une quantité à ajouter de manière dosée de liquide en fonction d'un ou de plusieurs paramètres de processus.

9. Dispositif selon au moins l'une quelconque des revendications précédentes,
- comprenant un dispositif de dosage de larves, qui est réalisé pour ajouter de manière dosée une quantité de larves et/ou d'œufs de larves définie en fonction d'un ou de plusieurs paramètres de processus et/ou en fonction d'une quantité à ajouter de manière dosée de larves et/ou d'œufs de larves définie par le dispositif de commande, et/ou
- comprenant un dispositif de dosage d'excréments d'animaux, qui est réalisé pour ajouter de manière dosée une quantité d'excréments d'animaux définie en fonction d'un ou de plusieurs paramètres de processus et/ou en fonction d'une quantité d'excréments d'animaux à ajouter de manière dosée définie par le dispositif de commande, et/ou
- comprenant un dispositif de dosage de liquide, qui est réalisé pour ajouter de manière dosée une quantité de liquide définie en fonction d'un ou de plusieurs paramètres de processus et/ou en fonction d'une quantité de liquide à ajouter de manière dosée définie par le dispositif de commande.

10. Dispositif selon au moins l'une quelconque des revendications précédentes, **caractérisé en ce qu'**au moins une zone de la ligne de convoyage est disposée dans un dispositif de séchage d'excréments ; et/ou
**caractérisé en ce que** des zones différentes de la ligne de convoyage sont disposées dans différents dispositifs de séchage d'excréments.

11. Dispositif selon au moins l'une quelconque des revendications précédentes, **caractérisé en ce que** la ligne de convoyage comprend l'une au-dessus de l'autre deux ou plusieurs lignes de convoyage partielles, qui présentent de préférence des sens de convoyage opposés et/ou sont formées de préférence par plusieurs convoyeurs à bande disposés les uns au-dessus des autres.

12. Dispositif selon au moins l'une quelconque des revendications précédentes, **caractérisé en ce que** le tronçon d'introduction d'excréments et le tronçon de mélange sont réalisés en tant qu'un tronçon individuel du tronçon de convoyage.

13. Installation de valorisation de résidus dans l'élevage d'animaux de rente,
comprenant un dispositif de valorisation de résidus dans l'élevage d'animaux de rente selon au moins l'une quelconque des revendications précédentes, un ou plusieurs premiers dispositifs de séchage d'excréments, et un deuxième dispositif de séchage d'excréments,
- dans laquelle des zones de la ligne de convoyage du dispositif de valorisation de résidus sont réalisées en tant que bandes de séchage du ou des plusieurs premiers dispositifs de séchage d'excréments et du deuxième dispositif de séchage d'excréments,
- dans laquelle le tronçon d'introduction de larves est disposé dans le premier dispositif de séchage d'excréments,
- dans laquelle le tronçon d'introduction d'excréments est disposé entre le premier et le deuxième dispositif de séchage d'excréments et/ou entre plusieurs premiers dispositifs de séchage d'excréments, et
- dans laquelle le tronçon de séparation de larves est disposé dans le deuxième dispositif de séchage d'excréments et/ou est installé en aval du deuxième dispositif de séchage d'excréments.

14. Installation de valorisation de résidus dans l'élevage d'animaux de rente selon la revendication précédente,
dans laquelle le tronçon de mélange est disposé entre le premier et le deuxième dispositif de séchage d'excréments et/ou entre plusieurs premiers dispositifs de séchage d'excréments.

15. Procédé de valorisation de résidus dans l'élevage d'animaux de rente, comprenant :
- le convoyage d'excréments d'animaux le long d'une ligne de convoyage,
- dans lequel la ligne de convoyage présente un tronçon d'introduction de larves, un tronçon d'introduction d'excréments et un tronçon de séparation de larves,
- dans lequel le tronçon de séparation de larves présente un dispositif de séparation avec une source de lumière (630) et un pont de larves (620a, 620b),
- l'ajout dosé de larves et/ou d'œufs de larves dans le tronçon d'introduction de larves,
- l'ajout dosé d'excréments d'animaux dans le tronçon d'introduction d'excréments en fonction d'un ou de plusieurs paramètres de processus,
- la séparation des larves dans le tronçon de séparation de larves,
**caractérisé par**
- l'éclairage du pont de larves au moyen de la source de lumière.
